# EUROPEAN PATENT APPLICATION

(11) **EP 4 772 648 A1**
(43) Date of publication of application: **08.07.2026**
(21) Application number: 24909387.3
(22) Date of filing: 23.02.2024
(51) Int. Cl.: C12P 19/40

(54) **METHOD FOR PREPARING PURINE NUCLEOSIDE**

(30) Priority: 27.12.2023 CN 202311820673
(71) Applicant: Tianjin Asymchem Biotechnology Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); LI, Mingji, Tianjin 300457 (CN); LI, Rui, Tianjin 300457 (CN); DING, Shimao, Tianjin 300457 (CN)
(74) Representative: HGF
(86) International application number: PCT/CN2024/078425
(87) International publication number: WO 2025/138415

(57) **Abstract**

Provided is a method for preparing purine nucleoside. The method includes catalyzing a uridine substrate and a substrate base by using a pyrimidine nucleoside phosphorylase and a purine nucleoside phosphorylase, and preparing and obtaining purine nucleoside. The uridine substrate includes uridine, 2'-deoxyuridine or 2'-amino-2'-deoxyuridine, and the substrate base includes a purine compound, wherein on the purine compound, R1 includes -NH₂, -OCH₃ or -Cl, and R2 includes -NH₂, -F or -Cl. The preparation method can solve the problem of low yield in the preparation of nucleoside compounds in the prior art, and is suitable for the field of enzyme catalysis.

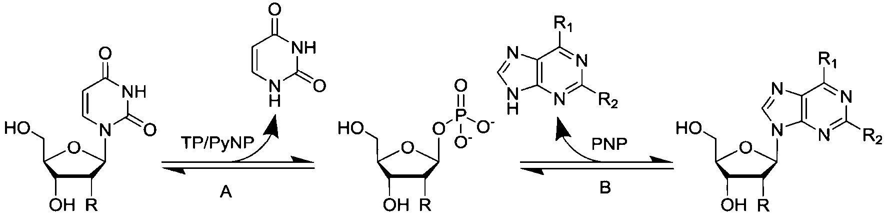

## Description

The present invention is based upon and claims the benefit of priority from Chinese Application No. 202311820673.7, filed on 27 December 2013, the disclosure of which is incorporated herein by reference in its entirety.

### Technical Field

The present invention relates to the field of enzyme catalysis, and in particular, to a method for preparing a purine nucleoside.

### Background

Nucleoside analogs, as pharmaceutical active ingredients such as pharmaceutical intermediates and receptor agonists, can take advantages in anti-virus and anti-tumor aspects, and can be used for treating various diseases. For example, fludarabin is the preferred drug for the treatment of chronic lymphocytic leukemia and low grade malignant lymphoma. Currently, nucleoside analogs are mainly synthesized by using a chemical method. However, in the synthesis process, a special chemical catalyst needs to be used, and various functional groups need to be protected and modified, and a plurality of complicated steps are required, which generally lead to disadvantages such as low yield, poor selectivity, and pollution to the environment.

In recent years, synthesis of nucleoside analogs by an enzymatic method has features such as mild conditions and good selectivity. In patent application WO 9412514 A1, guanosine is used as a substrate, and a key intermediate 2-fluoroadenosine of fludarabine can be synthesized via a multi-step reaction; however, the process thereof needs to use highly toxic hydrogen fluoride, and the reaction route is long and the yield is low, thereby limiting industrial application of the route.

### Summary

The main object of the present invention is to provide a method for preparing a purine nucleoside, so as to solve the problem in the prior art that the yield is low when a nucleoside compound is prepared with an enzyme synthesis method.

In order to achieve the described object, according to one aspect of the present invention, provided is a method for preparing a purine nucleoside. The method includes: catalyzing a uridine substrate and a substrate base with a pyrimidine nucleoside phosphorylase and a purine nucleoside phosphorylase, so as to obtain the purine nucleoside;
wherein the uridine substrate is a nucleoside as represented by formula I, and R includes -OH, -H or -NH₂; 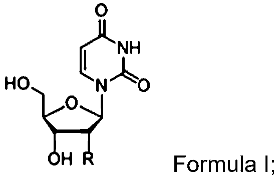
and the substrate base includes a purine compound as represented by formula II, wherein R₁ includes -NH₂, -OCH₃ or -Cl, and R₂ includes -NH₂, -F or -Cl;

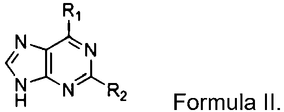

Further, R of the uridine substrate is -OH or -H, and at least one of R₁ or R₂ of the substrate base is halogen; and the substrate base includes one or more of 2-chloroadenine, 2-fluoroadenine, 2,6-dichloropurine, 6-chloro-2-fluoropurine, or 2-amino-6-chloropurine.

Further, R of the uridine substrate is -NH₂, and the substrate base includes one or more of 2-aminoadenine, 2-fluoroadenine, 2-chloroadenine, 2,6-dichloropurine, 2-amino-6-chloropurine, or 6-methoxyguanine.

Further, the pyrimidine nucleoside phosphorylase includes an amino acid sequence as shown in SEQ ID NO: 1 and/or SEQ ID NO: 2; or a protein having 70% or more identity to the amino acid sequence as shown in SEQ ID NO: 1 or SEQ ID NO:2 and having pyrimidine nucleoside phosphate activity; the purine nucleoside phosphorylase includes an amino acid sequence as shown in SEQ ID NO: 3; or a protein having 70% or more identity to the amino acid sequence as shown in SEQ ID NO: 3 and having purine nucleoside phosphorylase activity.

Further, R of the uridine substrate is -OH, and the pyrimidine nucleoside phosphorylase has an amino acid sequence as shown in SEQ ID NO: 2; preferably, R of the uridine substrate is -H or -NH₂, and the pyrimidine nucleoside phosphorylase has an amino acid sequence as shown in SEQ ID NO: 1 and/or SEQ ID NO: 2.

Further, R of the uridine substrate is -H, -NH₂ or -OH, and the purine nucleoside phosphorylase has an amino acid sequence as shown in SEQ ID NO: 3.

Further, the purine nucleoside includes one or more of 2-fluoroadenosine, 2-chloroadenosine, 2,6-dichloroadenosine, 6-chloro-2-fluoroadenosine, 6-chloroguanosine, 2'-fluorodeoxyadenosine, 2'-chlorodeoxyadenosine, 2,6-dichloropurine-2'-deoxyriboside, 6-chloro-2-fluoropurine-2'-deoxyriboside, 2-fluorodeoxyadenosine, 2-chlorodeoxyadenosine, 2,2'-diamino-2'-deoxyadenosine, 2-fluoro-2'-aminodeoxyadenosine, 2-chloro-2'-aminodeoxyadenosine, 2,6-dichloro-2'-aminodeoxyadenosine, 6-chloroguanine-2'-aminodeoxyadenosine, or 6-oxymethyl-2'-aminodeoxyguanosine.

Further, in the described method, the concentration of the uridine substrate ranges from 1 to 5000 mM, and the concentration of the substrate base ranges from 1 to 5000 mM.

Further, in the described preparation method, the reaction time ranges from 1 to 18 h; and preferably, the reaction temperature of the preparation method ranges from 40 to 80 °C.
In the technical solution of the present invention, by using a pyrimidine nucleoside phosphorylase and a purine nucleoside phosphorylase, a uridine substrate and a base substrate of specific structure are catalyzed in a one-step reaction to generate multiple purine nucleosides, thereby achieving amplified production, efficient synthesis within a shorter reaction time, and improved yields of the purine nucleosides.

### Brief Description of the Drawings

The accompanying drawings, which form a part of the present invention, are used to provide a further understanding of the present invention. The schematic embodiments of the present invention and the description thereof are used to explain the present invention, and do not form improper limits to the present invention. In the drawings:
Fig. 1 shows the reaction scheme of a preparation process according to an example of the present invention.
Fig. 2 shows an HPLC detection result chart of 2,6-dichloroadenosine according to Example 2 of the present invention.
Fig. 3 shows an HPLC detection result chart of 6-chloro-2-fluoroadenosine according to Example 2 of the present invention.
Fig. 4 shows an HPLC detection result chart of 6-chloroguanosine according to Example 2 of the present invention.
Fig. 5 shows an HPLC detection result chart of 2,6-dichloropurine-2'-deoxynucleoside according to Example 4 of the present invention.
Fig. 6 shows an HPLC detection result chart of 6-chloro-2-fluoropurine-2'-deoxynucleoside according to Example 4 of the present invention.
Fig. 7 shows an HPLC detection result chart of 2,6-dichloro-2'-aminodeoxyadenosine according to Example 5 of the present invention.
Fig. 8 shows an HPLC detection result chart of 6-chloroguanine-2'-aminodeoxyadenosine according to Example 5 of the present invention.
Fig. 9 shows an HPLC detection result chart of 6-oxymethyl-2'-aminodeoxyguanosine according to Example 5 of the present invention.

### Detailed Description of the Embodiments

It is important to note that the examples of the present invention and the characteristics in the examples can be combined under the condition of no conflicts. Hereinafter, the present invention will be described in detail with reference to examples.

As mentioned in the background art, methods for preparing purine nucleosides in the prior art all have problems such as high costs, long reaction times, low yields, etc., and are not conducive to preparation and subsequent use of purine nucleosides and analogs thereof. Accordingly, in the present invention, the inventors attempt to use a pyrimidine nucleoside phosphorylase and a purine nucleoside phosphorylase to catalyze a uridine substrate and a base substrate, and obtain purine nucleosides by a one-step catalytic method. In this reaction, due to the characteristics of the substrate and the base, the reaction time is short, the synthesis efficiency is high, and thus the yield of the obtained product is high, and industrial scale-up production can be performed. On this basis, the claimed solution of the present invention is proposed.

In a first typical embodiment of the present invention, provided is a method for preparing a purine nucleoside. The method includes: catalyzing a uridine substrate and a substrate base with a pyrimidine nucleoside phosphorylase and a purine nucleoside phosphorylase to prepare and obtain a purine nucleoside; wherein the uridine substrate is a nucleoside as represented by formula I, and R includes -OH, -H or -NH₂, and correspondingly, the uridine substrate is uridine, 2'-deoxyuridine or 2'-amino-2'-deoxyuridine; 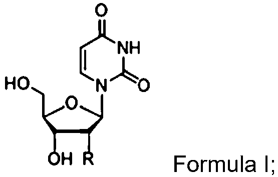 and the substrate base includes a purine compound as represented by formula II, wherein R₁ includes -NH₂, -OCH₃ or -Cl, and R₂ includes -NH₂, -F or -Cl;

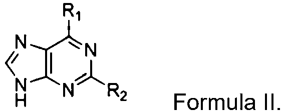

A nucleoside phosphorylase can catalyze a reversible phosphorylation reaction of the glycosidic bonds of nucleosides or deoxynucleosides to release a base and generate ribose-1-phosphate or deoxyribose-1-phosphate, and when another base exists, it combines with ribose-1-phosphate or deoxyribose-1-phosphate to form a new nucleoside. In the present invention, by an enzymatic method and by means of a pyrimidine nucleoside phosphorylase and a purine nucleoside phosphorylase, uridine, 2'-deoxyuridine and 2'-amino-2'-deoxyuridine can be converted into a variety of different purine nucleosides, and only a one-step reaction is needed. Compared with problems such as many steps and environmental pollution of current chemical synthesis methods, the preparation method in the present invention has a simple process and low costs, and is environmentally friendly, and the purine nucleoside can be synthesized by only a one-step reaction, with a short reaction time and high synthesis efficiency, which is of great significance and has a good application prospect to the production of industrialized synthetic nucleoside analogs.

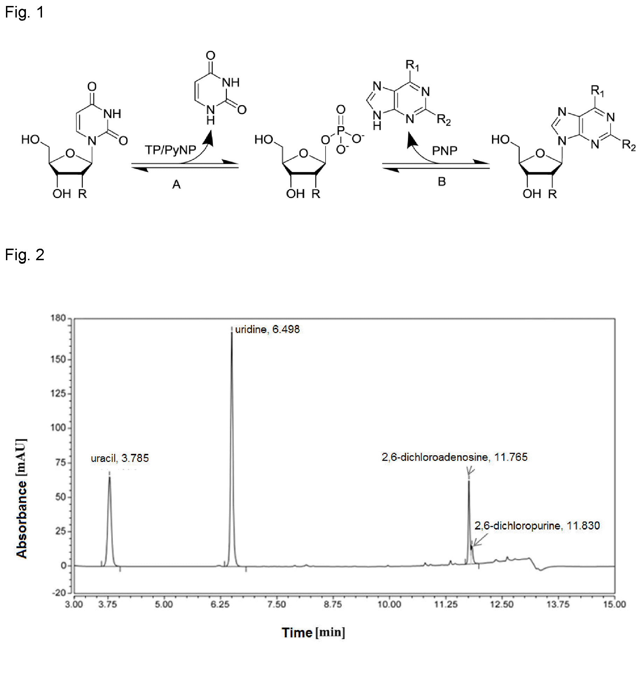

The pyrimidine nucleoside phosphorylase used in the present invention is derived from *Escherichia coli* and is a protein which has an amino sequence as shown in SEQ ID NO: 1, and the pyrimidine nucleoside phosphorylase is named TP; or *Geobacillus stearothermophilus*, and is a protein which has an amino sequence as shown in SEQ ID NO: 2, and the nucleoside phosphorylase is named PyNP. The purine nucleoside phosphorylase is derived from *Geobacillus thermoglucosidasius* and is a protein which has an amino sequence as shown in SEQ ID NO: 3, and the purine nucleoside phosphorylase is named PNP. The reaction scheme of the method above is shown in Fig. 1.

In the method, reaction A in Fig. 1 is carried out under the catalysis of TP or PyNP, the C-N bond between the pyrimidine group and the ribose group of the substrate nucleoside is broken, and a phosphate group is bonded to the ribose group to form a ribose phosphate intermediate. Under the action of PNP, the ribose phosphate intermediate undergoes reaction B in Fig. 1, so that the C-1 atom of the ribose group forms a C-N bond with the N-9 atom of a purine substrate base, and a purine nucleoside compound is produced. In the described reactions A and B, no additional auxiliary factors such as NADPH need to be added, and the reaction can be completed only with a substrate and a protein. The reaction is simple and does not need to be performed in living cells, thereby greatly reducing the reaction costs and improving efficiency. The enzymatic method performed by the combination of the two proteins can complete a catalytic reaction in a shorter catalytic time, has a high conversion rate of the substrate, and is also applicable to an amplified reaction, and can achieve an industrial production on a large scale.

The prior art discloses using a similar pyrimidine nucleoside phosphorylase and purine nucleoside phosphorylase to catalyze a similar reaction, and using a nucleoside and a base as substrates to catalyze the production of a new nucleoside. However, in the field of enzyme catalysis, substrates having different structures have a great influence on the efficiency of enzyme catalysis, and different substituents on the substrates usually have a great influence on the spatial structure, chemical properties, etc. of the substrate. According to the prior art, it is difficult to obtain the motivation regarding for different types of substrates, which method needs to be used to achieve a similar reaction, and it is more difficult to predict the effect produced by the catalysis of different enzymes.

In a preferred example, R of the uridine substrate is -OH or -H, and at least one of R₁ or R₂ of the substrate base is a halogen; and the substrate base includes one or more of 2-chloroadenine, 2-fluoroadenine, 2,6-dichloropurine, 6-chloro-2-fluoropurine, or 2-amino-6-chloropurine.

In a preferred example, R of the uridine substrate is -NH₂, and the substrate base includes one or more of 2-aminoadenine, 2-fluoroadenine, 2-chloroadenine, 2,6-dichloropurine, 2-amino-6-chloropurine, or 6-methoxyguanine.

When synthesizing a halogen-containing nucleoside product, if the method in the prior art is used, large amounts of toxic halogenides are consumed, which results in stringent operation requirements for the preparation process and environmental pollution. In addition, in the present invention, by using a non-toxic substrate base carrying a halogen substituent, a purine nucleoside product carrying a halogen substituent can be synthesized in only a one-step reaction. This process is easy to operate, avoids the use of toxic halogenides, is safe to operate, and is environmentally friendly.

In a preferred example, the pyrimidine nucleoside phosphorylase includes an amino acid sequence as shown in SEQ ID NO: 1 and/or SEQ ID NO: 2; or a protein having 70% or more (including but not limited to 80%, 85%, 90%, 95%, 98%, 99%, 99.5% or 99.9%) identity to the amino acid sequence as shown in SEQ ID NO: 1 or SEQ ID NO: 2 and having pyrimidine nucleoside phosphorylation activity; the amino acid sequence of the purine nucleoside phosphorylase as shown in SEQ ID NO: 3; or a protein having 70% or more (including but not limited to 80%, 85%, 90%, 95%, 98%, 99%, 99.5%, or 99.9%) identity to the amino acid sequence as shown in SEQ ID NO: 3 and having purine nucleoside phosphorylation activity.

Identity in the present invention refers to "identity" between amino acid sequences or nucleic acid sequences, i.e. the sum of the ratios of amino acid residues or nucleotides of identical species in an amino acid sequence or nucleic acid sequence. The identity of an amino acid sequence or nucleic acid sequence may be determined using BLAST (Basic Local Alignment Search Tool), FASTA, and other alignment programs.

For proteins having 70%, 75%, 80%, 85%, 90%, 95%, or 99% or more (for example, 85%, 86%, 87%, 88%, 89%, 90%, 91%, 92%, 93%, 94%, 95%, 96%, 97%, 98%, 98.5%, 99%, 99.5%, 99.6%, 99.7%, 99.8% or more, or even 99.9% or more) identity and the same function, the active site, active pocket, active mechanism, and protein structure thereof are all very likely the same as those of the protein provided by the described sequence.

As used herein, amino acid residues are abbreviated as follows: Alanine (Ala; A), Asparagine (Asn; N), Aspartic acid (Asp; D), arginine (Arg; R), cysteine (Cys; C), glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V).

With regard to rules such as substitution and replacement, in general, amino acids having similar properties have similar effect after replacement. For example, in the described homologous proteins, conservative amino acid substitutions may occur. "Conservative amino acid substitutions" include, but are not limited to:
hydrophobic amino acids (Ala, Cys, Gly, Pro, Met, Val, Ile, and Leu) being substituted by other hydrophobic amino acids;
hydrophobic amino acids having a large side chain (Phe, Tyr, and Trp) being substituted by other hydrophobic amino acids having a large side chain;
amino acids having a positively charged side chain (Arg, His, and Lys) being substituted by other amino acids having a positively charged side chain; and
amino acids having an uncharged polar side chain (Ser, Thr, Asn, and Gin) being substituted by other amino acids having an uncharged polar side chain.

A person skilled in the art would also have been able to perform conservative replacements on amino acids according to amino acid replacement rules well known to a person skilled in the art, such as "blosum62 scoring matrix" in the prior art.
SEQ ID NO: 1: 
MFLAQEIIRKKRDGHALSDEEIRFFINGIRDNTISEGQIAALAMTIFFHDMTMPERVSLTMAMRD
SGTVLDWKSLHLNGPIVDKHSTGGVGDVTSLMLGPMVAACGGYIPMISGRGLGHTGGTLDKLESI
PGFDIFPDDNRFREIIKDVGVAIIGQTSSLAPADKRFYATRDITATVDSIPLITASILAKKLAEGLDALVM
DVKVGSGAFMPTYELSEALAEAIVGVANGAGVRTTALLTDMNQVLASSAGNAVEVREAVQFLTGE
YRNPRLFDVTMALCVEMLISGKLAKDDAEARAKLQAVLDNGKAAEVFGRMVAAQKGPTDFVENYA
KYLPTAMLTKAVYADTEGFVSEMDTRALGMAVVAMGGGRRQASDTIDYSVGFTDMARLGDQVDG
QRPLAVIHAKDENNWQEAAKAVKAAIKLADKAPESTPTVYRRISE.
SEQ ID NO: 2: 
MRMVDLIEKKRDGHALTKEEIQFIIEGYTKGDIPDYQMSALAMAIFFRGMNEEETAELTMAMVH
SGDTIDLSRIEGIKVDKHSTGGVGDTTTLVLGPLVASVGVPVAKMSGRGLGHTGGTIDKLESVPGF
HVEITNDEFIDLVNKNKIAVVGQSGNLTPADKKLYALRDVTATVNSIPLIASSIMSKKIAAGADAIVLDV
KTGVGAFMKDLNDAKALAKAMVDIGNRVGRKTMAIISDMSQPLGYAIGNALEVKEAIDTLKGEGPE
DFQELCLVLGSHMVYLAEKASSLEEARHMLEKAMKDGSALQTFKTFLAAQGGDASVVDDPSKLP
QAKYIIELEAKEDGYVSEIVADAVGTAAMWLGAGRATKESTIDLAVGLVLRKKVGDAVKKGESLVTIY
SNREQVDDVKQKLYENIRISATPVQAPTLIYDKIS.
SEQ ID NO: 3:

MSIHIEAKQQEIAEKILLPGDPLRAQYIAETFLEGATCYNRVRGMLGFTGTYKGHRISVQGTGM
GVPSISIYVNELIQSYHVQTLIRVGTCGAIQKDVNVRDVILAMSASTDSNMNRLTFRGRDYAPTANF
ALLRTAYEVGAEKGLPLKVGSVFTADMFYNDEPDWETWARYGVLAVEMETAALYTLAAKFGRKAL
SVLTVSDHILTGEETTAQERQTTFNDMIEVALETAIRVE.

In a preferred example, R of the uridine substrate is -OH, and the pyrimidine nucleoside phosphorylase has an amino acid sequence as shown in SEQ ID NO: 2; and preferably, R of the uridine substrate is -H or -NH₂, and the pyrimidine nucleoside phosphorylase has an amino acid sequence as shown in SEQ ID NO: 1 and/or SEQ ID NO: 2.

In a preferred example, R of the uridine substrate is -OH, -H, or -NH₂, and the purine nucleoside phosphorylase has an amino acid sequence as shown in SEQ ID NO: 3; and preferably, R of the uridine substrate is -OH, and the purine nucleoside phosphorylase has an amino acid sequence as shown in SEQ ID NO: 3.

The active centers of different enzymes may have different binding sites for different substrates; therefore, when R of the uridine substrate is -H or -NH₂, the pyrimidine nucleoside phosphorylase can be TP or PyNP, and both TP and PyNP can bind to the two substrates for a catalytic reaction.

In the catalytic system including TP, PyNP, and PNP, the three described proteins can exist in various forms, such as purified proteins, crude enzyme solutions, or immobilized enzymes, and can all catalyze the synthesis of purine nucleosides. A gene capable of expressing the described proteins is cloned into a host cell, protein expression is induced, and the host cell is lysed to obtain a crude enzyme solution containing the target protein. The crude enzyme solution is simple to prepare, has good catalytic activity, and can reduce the production costs of the catalytic reaction.In a preferred example, the purine nucleoside includes 2-fluoroadenosine, 2-chloroadenosine, 2,6-dichloroadenosine, 6-chloro-2-fluoroadenosine, 6-chloroguanosine, 2-fluorodeoxyadenosine, 2-chlorodeoxyadenosine, 2,6-dichloropurine-2'-deoxyriboside, 6-chloro-2-fluoropurine-2'-deoxyriboside, 2-fluorodeoxyadenosine, 2-chlorodeoxyadenosine, 2,2'-diamino-2'-deoxyadenosine, 2-fluoro-2'-aminodeoxyadenosine, 2-chloro-2'-aminodeoxyadenosine, 2,6-dichloro-2'-aminodeoxyadenosine, 6-chloroguanine-2'-aminodeoxyadenosine, or 6-oxymethyl-2'-aminodeoxyguanosine.

Different substrate nucleosides and different substrate bases have different biochemical properties; therefore, during synthesis, TP, PyNP, and PNP can all achieve an enzyme catalyzed reaction on the described different substrate nucleosides and substrate bases, thereby preparing the described various purine nucleosides.

In a preferred example, the concentration of the uridine substrate in the preparation method is 1-5000 mM, and the concentration of the substrate base is 1-5000 mM.

The concentration of the described uridine substrate includes, but is not limited to, 1, 2, 5, 10, 15, 20, 25, 30, 35, 40, 45, 50, 55, 60, 65, 70, 75, 80, 85, 90, 100, 150, 200, 250, 300, 320, 400, 500, 600, 800, 1000, 1500, 2000, 2500, 3000, 4000, or 5000 mM. The concentration of the substrate base includes, but is not limited to, 1, 2, 3, 4, 5, 6, 7, 8, 9, 10, 11, 12, 13, 14, 15, 16, 17, 18, 19, 20, 50, 80, 100, 150, 200, 300, 400, 500, 600, 800, 1000, 15000, 2000, 2500, 3000, 4000, or 5000 mM.

In a preferred example, the catalytic time of the enzyme catalysis is 1-18 h.

The described enzyme catalysis time includes, but is not limited to, 1, 2, 3, 4, 5, 6, 7, 8, 9, 10, 11, 12, 13, 14, 15, 16, 17, or 18 h.

In a preferred example, the catalytic temperature for enzyme catalysis is 40-80°C.

The described catalytic temperature includes, but is not limited to, 40, 50, 60, 70, or 80°C.

In the described suitable catalytic temperature and catalytic time, the enzyme catalytic reaction can be completed, and the conversion rate of the substrate base, i.e. the reaction yield, is relatively high. There is also no need to supplement an enzyme or other reagents during the reaction, and the reaction can be completed through one-step catalysis, which is suitable for application in industrial scale-up production.

### Example 1

### 1. Strain construction

The pyrimidine nucleoside phosphorylase used in the present invention was derived from *Escherichia coli* and was a protein which has the amino acid sequence as shown in SEQ ID NO: 1, and the pyrimidine nucleoside phosphorylase was named TP; or *Geobacillus stearothermophilus*, and was a protein which has the amino acid sequence as shown in SEQ ID NO: 2, and the pyrimidine nucleoside phosphorylase was named PyNP. The purine nucleoside phosphorylase was derived from *Geobacillus thermoglucosidasius* and was a protein which has the amino acid sequence as shown in SEQ ID NO: 3, and the purine nucleoside phosphorylase was named PNP.

In the described method, a purine nucleoside was efficiently synthesized using two nucleoside phosphatases of different sources in a one-step method, and therefore the production costs can be reduced, and the method can be applied to large-scale industrial production of related products. The described substrate includes a substrate nucleoside and a substrate base, which may be a natural nucleoside or base structure, and may also have an artificially modified similar structure.

DNA sequences encoding the described three enzymes were obtained after codon optimization, the DNA sequence encoding TP being SEQ ID NO: 4; the DNA sequence encoding PyNP being SEQ ID NO: 5; and the DNA sequence encoding PNP being SEQ ID NO: 6. Each was cloned into an expression vector pET28a(+). The resulting plasmid was transferred into the E. coli BL21(DE3) host competent, resulting in a monoclonal strain.
SEQ ID NO: 4: 
ATGTTTCTGGCGCAGGAAATTATTCGCAAGAAACGCGATGGCCATGCGCTGAGCGATGAA
GAAATTCGCTTTTTCATTAACGGCATCCGCGATAACACCATTAGCGAAGGCCAGATTGCGGCGC
TGGCGATGACTATTTTCTTTCATGATATGACCATGCCGGAACGCGTGAGCCTGACGATGGCGAT
GCGTGATAGCGGCACCGTTCTGGATTGGAAAAGCCTGCATCTGAACGGCCCGATTGTGGATAA
ACATAGCACCGGCGGCGTGGGCGATGTGACCAGCTTAATGTTAGGCCCGATGGTGGCGGCGT
GCGGCGGTTATATTCCGATGATTAGCGGTCGCGGCCTGGGCCATACCGGTGGTACCTTAGATA
AACTGGAAAGCATTCCGGGCTTTGATATTTTTCCGGATGATAACCGCTTTCGCGAAATTATTAAA
GACGTGGGCGTGGCGATTATTGGCCAGACCAGCAGCCTGGCGCCGGCGGATAAACGTTTTTA
 
TGCGACCCGCGATATTACCGCGACCGTGGATAGCATTCCGCTGATTACCGCGAGCATTCTGGC
GAAAAAGCTGGCGGAAGGCCTGGATGCGCTGGTGATGGATGTGAAAGTGGGCAGCGGCGCG
TTTATGCCGACCTATGAACTGAGCGAAGCGCTGGCGGAAGCGATTGTGGGCGTTGCGAATGG
CGCGGGCGTACGTACCACCGCACTGTTAACCGATATGAACCAGGTGCTGGCGAGCAGCGCGG
GCAATGCGGTTGAAGTTCGTGAAGCGGTTCAATTTCTGACCGGCGAATATCGCAACCCGCGCC
TGTTTGATGTGACGATGGCGCTGTGCGTGGAAATGCTGATTAGCGGCAAACTGGCGAAAGATG
ATGCGGAAGCGCGTGCCAAACTGCAGGCGGTTTTAGATAACGGCAAAGCGGCGGAAGTGTTT
GGCCGCATGGTGGCGGCACAAAAAGGCCCTACTGATTTTGTGGAAAACTATGCGAAATATCTG
CCGACCGCGATGCTGACCAAAGCGGTGTATGCGGATACCGAAGGCTTTGTGAGCGAAATGGA
TACCCGCGCCCTGGGCATGGCGGTTGTTGCGATGGGTGGTGGTCGTCGTCAAGCGAGCGATA
CCATTGATTATAGCGTGGGCTTTACCGATATGGCGCGTCTGGGCGATCAGGTGGATGGCCAAC
GTCCTTTAGCGGTGATTCATGCGAAAGATGAAAACAACTGGCAGGAAGCGGCGAAAGCGGTG
AAAGCGGCGATTAAACTGGCGGATAAAGCGCCGGAAAGCACCCCGACCGTTTATCGCCGTATT
AGCGAATAA.
SEQ ID NO: 5: 
ATGCGCATGGTGGATCTGATTGAAAAAAAACGCGATGGCCATGCGCTGACCAAAGAAGAA
ATTCAGTTTATTATTGAAGGCTATACCAAAGGCGATATTCCGGATTATCAGATGAGCGCGCTGGC
GATGGCGATTTTTTTTCGCGGCATGAACGAAGAAACCGCGGAACTGACGATGGCGATGGT
GCATAGCGGCGATACCATTGATCTGAGCCGCATTGAAGGCATTAAAGTGGATAAACATAGTACC
GGAGGCGTAGGTGATACCACCACCCTTGTACTGGGCCCGCTTGTGGCTAGTGTGGGCGTGCC
GGTGGCGAAAATGAGTGGCCGCGGCCTGGGCCATACCGGCGGCACCATTGATAAACTGGAAA
GCGTGCCGGGCTTTCATGTGGAAATTACCAACGATGAATTTATTGATCTGGTGAACAAAAACAA
AATTGCGGTGGTGGGTCAGAGCGGCAACCTGACCCCGGCGGATAAAAAACTGTATGCGCTGC
GCGATGTGACCGCGACCGTGAACAGCATTCCGCTGATTGCGAGCAGCATTATGAGCAAAAAAA
TTGCGGCGGGCGCGGATGCGATTGTGCTGGATGTGAAAACCGGCGTGGGCGCGTTTATGAAG
GATCTGAACGATGCGAAAGCGCTGGCGAAAGCGATGGTGGATATTGGCAACCGCGTGGGCCG
CAAAACGATGGCGATTATTAGCGATATGAGTCAGCCGCTGGGCTATGCGATTGGCAACGCGCT
GGAAGTGAAAGAAGCGATTGATACCCTGAAAGGCGAAGGCCCGGAAGATTTTCAAGAACTGT
GCCTCGTGCTGGGCAGCCACATGGTGTATCTGGCGGAAAAAGCGAGCAGCCTGGAAGAAGC
GCGCCACATGCTGGAAAAAGCGATGAAAGATGGCAGCGCGCTGCAGACCTTTAAAACCTTTCT
GGCGGCGCAAGGCGGCGATGCGAGCGTGGTGGATGATCCGAGCAAACTGCCGCAAGCGAAA
TATATTATTGAACTGGAAGCGAAAGAAGATGGCTATGTGAGCGAAATTGTGGCGGATGCGGTG
GGCACCGCGGCGATGTGGCTGGGCGCGGGCCGCGCGACCAAAGAAAGCACCATTGATCTGG
CGGTGGGCCTGGTGCTGCGCAAAAAAGTGGGCGATGCGGTGAAAAAAGGCGAAAGCCTGGT
 
GACCATTTATAGCAACCGCGAACAAGTGGATGATGTGAAACAGAAACTGTATGAAAACATTCGC
ATTAGCGCGACCCCGGTGCAAGCGCCGACCCTGATTTATGATAAAATTAGCTAA.
SEQ ID NO: 6:

ATGAGCATTCATATTGAAGCGAAACAGCAGGAAATTGCGGAAAAGATTCTGCTGCCGGGC
GATCCGTTACGTGCGCAATATATTGCGGAAACCTTTCTGGAAGGCGCGACCTGCTATAACCGC
GTGCGCGGTATGCTGGGCTTTACCGGTACCTATAAAGGCCATCGCATTAGCGTGCAGGGCACC
GGCATGGGCGTTCCGTCAATTAGCATTTATGTGAACGAACTGATTCAGAGCTATCACGTGCAGA
CCCTGATTCGCGTGGGCACCTGTGGCGCAATTCAGAAAGATGTGAACGTGCGCGATGTGATT
CTGGCGATGAGCGCGAGCACCGATAGCAACATGAACCGCCTGACCTTTCGCGGCCGTGATTA
TGCGCCTACCGCGAATTTTGCGCTGCTGCGCACTGCGTATGAAGTGGGCGCGGAAAAAGGCC
TGCCGCTGAAAGTGGGCAGCGTGTTTACCGCGGATATGTTTTATAACGATGAACCGGATTGGG
AAACCTGGGCCCGCTATGGCGTTCTGGCGGTGGAAATGGAAACCGCGGCGTTATATACCCTG
GCGGCGAAATTTGGCCGCAAAGCGCTGAGCGTGCTGACCGTGAGCGATCATATTCTGACCGG
CGAAGAAACCACCGCGCAGGAACGCCAAACCACCTTTAACGATATGATTGAAGTGGCGCTGG
AAACCGCGATTCGCGTGGAATAA.

### 2. Protein expression

Escherichia coli strains expressing TP, PyNP and PNP were inoculated into tubes, respectively, incubated at 37°C for 16 h, and then inoculated into 2L shake flasks containing 500 mL of Luria-Bertani medium at an inoculation amount of 1%. When cultured at 37°C until the OD₆₀₀ was 0.6, isopropyl-β-D-thiogalactoside at a final concentration of 0.1 M was added, and cultured at 20°C for 18 h. After the culture, the strain was collected by centrifugation of the bacterial solution at 7000 rpm for 10 min for use.

### 3. Preparation of enzyme solution

0.1 g of bacterial sludge was weighed, 1 mL of potassium phosphate buffer solution with pH 7.5 was added. After shaking and mixing evenly, the strain suspension was disrupted with an ultrasonic disruptor at a power of 30% for 5 min.

### 4. HPLC detection method

The chromatographic column was Atlantis T3 Column, with 4.6 mm x 150 mm. The mobile phase was methanol containing 0.1 v/v% trifluoroacetic acid (TFA). The flow rate was 1 mL/min. The column temperature was 40°C. The UV detector had a detection wavelength of 254 nm and a detection duration of 15 min.

### Example 2 Purine nucleoside synthesis with uridine as substrate

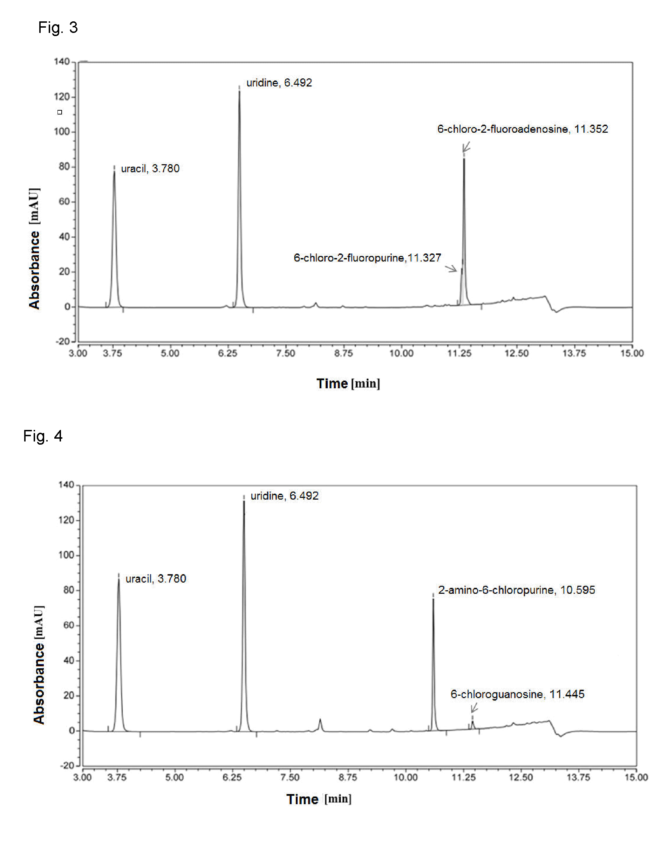

1 mL of reaction system was prepared in a 2 mM phosphate buffer (pH 7.5), and the reaction system included 20 mM uridine, 10 mM purine analog, 4.8 mg of a PyNP enzyme solution prepared from the bacterial sludge, and 1.9 mg of a PNP enzyme solution prepared from the bacterial sludge. The reaction was performed at 60°C for 18 h. After the reaction was finished, 1 mL of DMSO was added for HPLC detection. The detection results of products 2,6-dichloroadenosine, 2,6-dichloroadenosine and 6-chloroguanosine were shown in figures 2, 3 and 4. The conversion results were shown in Table 1, wherein the reaction conversion of 2,6-dichloropurine can reach 80% or more.

**Table 1**

| Substrate base | Product | Purine conversion |
|---|---|---|
| 2,6-dichloropurine | 2,6-dichloroadenosine | +++++ |
| 6-chloro-2-fluoropurine | 6-chloro-2-fluoroadenosine | +++++ |
| 2-amino-6-chloropurine | 6-chloroguanosine | + |

| | | |
|---|---|---|
| Note: "+" represents a conversion of 5-20%, and "+++++" represents a conversion of 70-90%. The structural formulas of 2,6-dichloroadenosine, 6-chloro-2-fluoroadenosine and 6-chloroguanosine were shown below: | | |

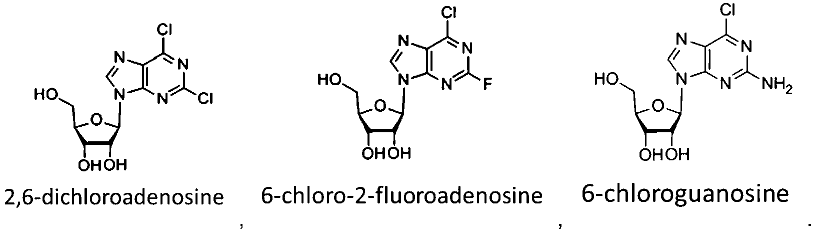

1 mL of reaction system was prepared in a 2 mM phosphate buffer (pH 7.5), and the reaction system included 2 mM uridine, 1 mM purine analog, 0.1 mg of a PyNP enzyme solution prepared from the bacterial sludge, and 0.08 mg of a PNP enzyme solution prepared from the bacterial sludge. The reaction was performed at 60°C for 18 h. After the reaction was finished, 1 mL of DMSO was added for HPLC detection. The conversion results were shown in Table 2, wherein the reaction conversion of 2-fluoroadenine can reach 90% or more.

**Table 2**

| Substrate base | Product | Purine conversion |
|---|---|---|
| 2-fluoroadenine | 2-fluoroadenosine | ++++++ |
| 2-chloroadenine | 2-chloroadenosine | +++++ |

| | | |
|---|---|---|
| Note: "+++++" represents a conversion of 70-90%, and "++++++" represents a conversion of >90%. The structural formulas of 2-fluoroadenosine and 2-chloroadenosine were shown below: | | |

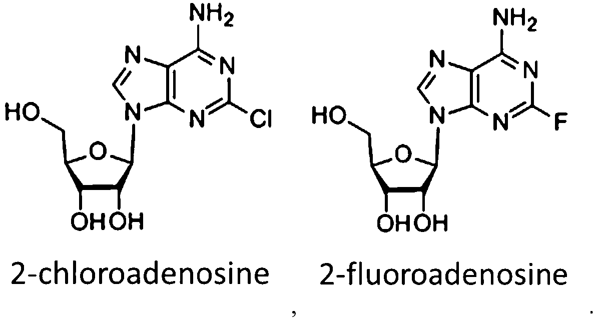

### Example 3 Amplified reaction with uridine as substrate

1 mL of reaction system was prepared in a 2 mM phosphate buffer (pH 7.5), and the reaction system included 320 mM uridine, 200 mM purine analog (2-fluoroadenine or 2-chloroadenine), 19.5 mg of a PyNP enzyme solution prepared from the bacterial sludge, and 15.3 mg of a PNP enzyme solution prepared from the bacterial sludge. The reaction was performed at 60°C for 18 h. After the reaction was finished, 1 mL of DMSO was added for HPLC detection. The conversion results showed that the reaction conversion of 2-fluoroadenine was 80% or more, and the reaction conversion of 2-chloroadenine was 80% or more.

### Example 4 Synthesis of purine nucleoside by using 2'-deoxyuridine as substrate

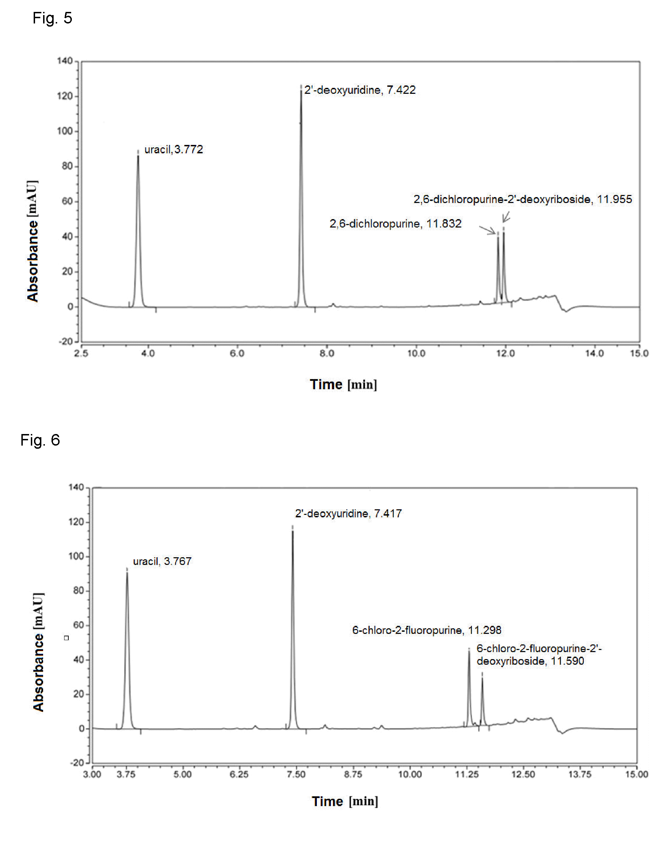

1 mL of reaction system was prepared in a 2 mM phosphate buffer (pH 7.5), and the reaction system included 20 mM 2'-deoxyuridine, 10 mM purine analog, 4.6 mg of a TP or PyNP enzyme solution prepared from the bacterial sludge, and 1.9 mg of a PNP enzyme solution prepared from the bacterial sludge. The reaction was performed at 60°C for 18 h. After the reaction was finished, 1 mL of DMSO was added for HPLC detection. The detection results of products 2,6-dichloropurine-2'-deoxynucleoside and 6-chloro-2-fluoropurine-2'-deoxynucleoside were shown in figures 5 and 6. The conversion results were shown in Table 3, wherein the reaction conversion of 2,6-dichloropurine can reach 50% or more.

**Table 3**

| Substrate base | Product | Purine conversion |
|---|---|---|
| 2,6-dichloropurine | 2,6-dichloropurine-2'-deoxynucleoside | ++++ |
| 6-chloro-2-fluoropurine | 6-chloro-2-fluoropurine-2'-deoxynucleoside | +++ |

| | | |
|---|---|---|
| Note: "+++" represents a conversion of 30-50% (excluding endpoint value of 50%), and "++++" represents a conversion of 50-70%. The structural formulas of 2,6-dichloropurine-2'-deoxynucleoside and 6-chloro-2-fluoropurine-2'-deoxynucleoside were shown below. | | |

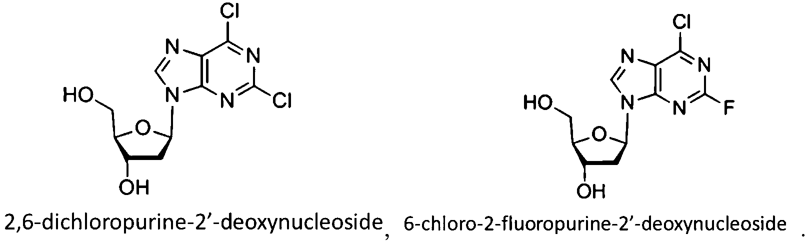

1 mL of reaction system was prepared in 2 mM phosphate buffer (pH 7.5), and the reaction system included 4 mM 2'-deoxyuridine, 1 mM purine analog, 0.9 mg of a TP or PyNP enzyme solution prepared from the bacterial sludge, and 1.9 mg of a PNP enzyme solution prepared from the bacterial sludge. The reaction was performed at 60°C for 18 h. After the reaction was finished, 1 mL of DMSO was added to perform HPLC detection. The conversion results were shown in Table 4, wherein the reaction conversion of 2-fluoroadenine can reach 60% or more.

**Table 4**

| Substrate base | Product | Purine conversion |
|---|---|---|
| 2-fluoroadenine | 2'-fluorodeoxyadenosine | ++++ |
| 2-chloroadenine | 2'-chlorodeoxyadenosine | +++ |

| | | |
|---|---|---|
| Note: "+++" represents a conversion of 30-50% (excluding endpoint value of 50%), and "++++" represents a conversion of 50-70%. The formulas of 2-fluorodeoxyadenosine and 2-chlorodeoxyadenosine were shown below: | | |

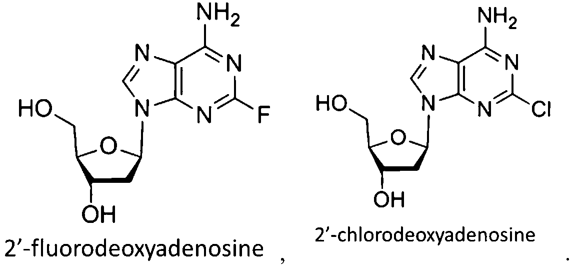

### Example 5 Synthesis of purine nucleoside by using 2'-amino-2'-deoxyuridine as substrate

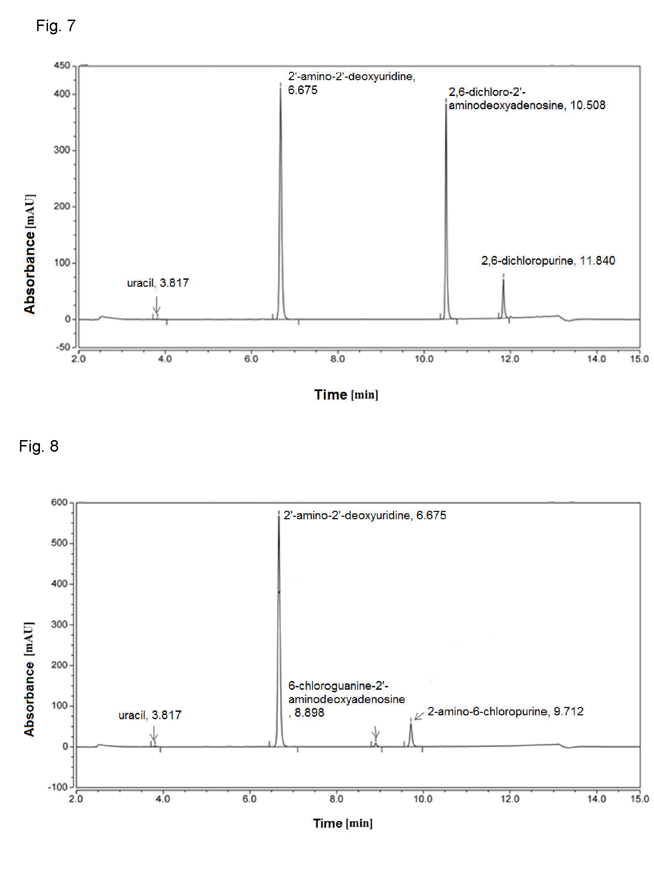

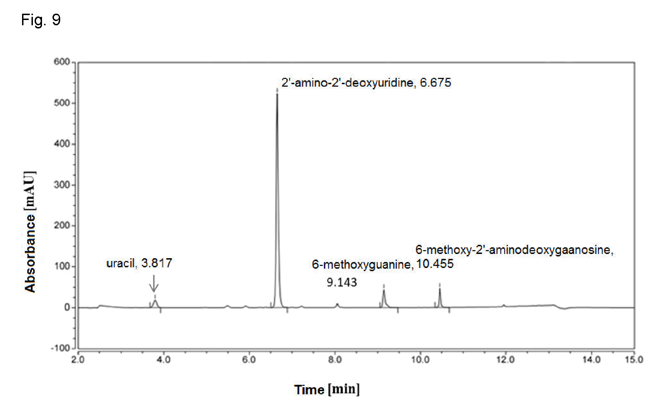

1 mL of reaction system was prepared in 2 mM phosphate buffer (pH 7.5), and the reaction system included 40 mM 2-amino-2-deoxyuridine, 20 mM purine analog, 9.8 mg of a TP or PyNP enzyme solution prepared from the bacterial sludge, and 3.8 mg of a PNP enzyme solution prepared from the bacterial sludge. The reaction was performed at 60°C for 18 h. After the reaction was finished, 1 mL of DMSO was added for HPLC detection. The detection results of products 2,6-dichloro-2'-aminodeoxyadenosine, 6-chloroguanine-2'-aminodeoxyadenosine and 6-oxymethyl-2'-aminodeoxyguanosine were shown in figures 7, 8 and 9. The conversion results were shown in Table 5, wherein the reaction conversion of 2,6-dichloropurine can reach 80% or more.

**Table 5**

| Substrate base | Product | Purine conversion |
|---|---|---|
| 2,6-dichloropurine | 2,6-dichloro-2'-aminodeoxyadenosine | +++++ |
| 2-amino-6-chloropurine | 6-chloroguanine-2'-aminodeoxyadenosine | + |
| 6-methoxyguanine | 6-oxymethyl-2'-aminodeoxyguanosine | +++ |

| | | |
|---|---|---|
| Note: "+" represents a conversion of 5-20%, "+++" represents a conversion of 30-50%, and "+++++" represents a conversion of 70-90%. The formulas of 2,6-dichloro-2'-aminodeoxyadenosine, 6-chloroguanine-2'-aminodeoxyadenosine and 6-oxymethyl-2'-aminodeoxyguanosine were shown below: | | |

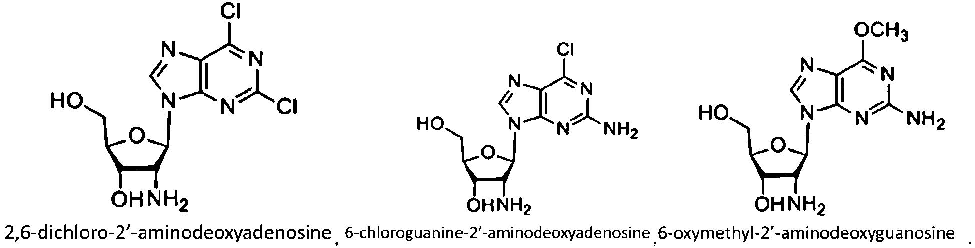

1 mL of reaction system was prepared in 2 mM phosphate buffer (pH 7.5), and the reaction system included 2 mM 2'-amino-2-deoxyuridine, 1 mM purine analog, 2.5 mg of a TP or PyNP enzyme solution prepared from the bacterial sludge, and 0.1 mg of a PNP enzyme solution prepared from the bacterial sludge. The reaction was performed at 60°C for 18 h. After the reaction was finished, 1 mL of DMSO was added for HPLC detection. The conversion results were shown in Table 6, wherein the reaction conversion of 2-aminopurine can reach 95% or more.

**Table 6**

| Substrate base | Product | Purine conversion |
|---|---|---|
| 2-aminoadenine | 2,2'-diamino-2'-deoxyadenosine | ++++++ |
| 2-fluoroadenine | 2-fluoro-2'-aminodeoxyadenosine | ++++ |
| 2-chloroadenine | 2-chloro-2'-aminodeoxyadenosine | ++++ |

| | | |
|---|---|---|
| Note: "++++" represents a conversion of 50-70%, and "++++++" represents a conversion of >90%. The formulas of 2,2'-diamino-2'-deoxyadenosine, 2-fluoro-2'-aminodeoxyadenosine and 2-chloro-2'-aminodeoxyadenosine were shown below. | | |

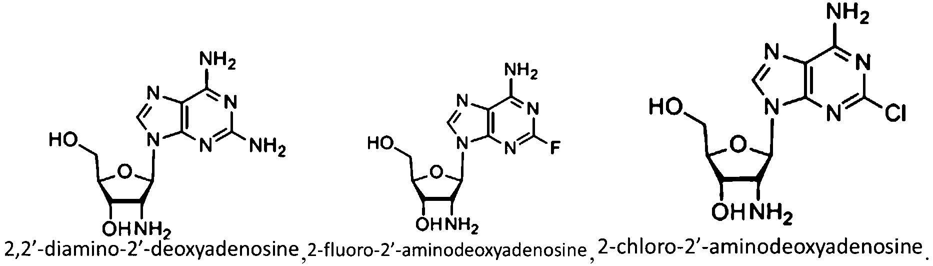

### 2,2'-diamino-2'-deoxyadenosine 2-fluoro-2'-aminodeoxyadenosine 2-chloro-2'-aminodeoxyadenosine

When the substrate base is 6-chloro-2-fluoropurine, nearly no purine conversion could be detected.

From the description above, it is evident that the described examples of the present invention achieve the following technical effects: by using the described enzyme catalysis method, within a short catalytic period, uridine nucleosides such as uridine, 2'-deoxyuridine, or 2'-amino-2'-deoxyuridine and substrate bases such as 2-chloroadenine, 2-fluoroadenine, 2,6-dichloropurine, 6-chloro-2-fluoropurine, 2-amino-6-chloropurine, 2-aminoadenine, or 6-methoxyguanine can be used as substrates to synthesize purine nucleoside products, enabling amplified production, and purine nucleosides of specific structures can be efficiently synthesized in a relatively short reaction time. Purine nucleosides of such structures are difficult to prepare by means of a biocatalysis method in the prior art, and can only be prepared by means of a chemical synthesis method. The chemical synthesis method has complex steps, is time-consuming, and has poor selectivity. The use of organic reagents tends to cause environmental pollution. By means of the technical solution of the present invention, purine nucleosides of specific structures can be prepared in a one-step reaction. The method has simple steps, uses fewer reagents, is environmentally friendly and highly efficient, and is applicable to industrialization for amplified production.The description above is only the preferred examples of the present invention, and is not intended to limit the present invention. For those skilled in the art, the present invention may have various modifications and variations. Any modifications, equivalent replacements, improvements and the like made within the spirit and principle of the present invention shall fall within the protection scope of the present invention.

## Claims

1. A method for preparing a purine nucleoside, wherein the method comprises:
catalyzing a uridine substrate and a substrate base with a pyrimidine nucleoside phosphorylase and a purine nucleoside phosphorylase, and obtaining the purine nucleoside;
wherein the uridine substrate is a nucleoside as represented by formula I, wherein R comprises -OH, -H or -NH₂; 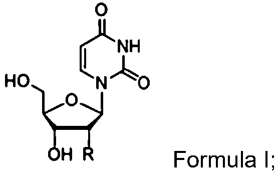
and the substrate base comprises a purine compound as represented by formula II, wherein R₁ comprises -NH₂, -OCH₃ or -Cl, and R₂ comprises -NH₂, -F or -Cl;

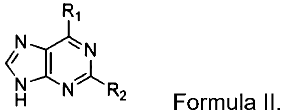

2. The method of claim 1, wherein the R of the uridine substrate is -OH or -H, and at least one of the R₁ or the R₂ of the substrate base is halogen; and
the substrate base comprises one or more of 2-chloroadenine, 2-fluoroadenine, 2,6-dichloropurine, 6-chloro-2-fluoropurine, or 2-amino-6-chloropurine.

3. The method of claim 1, wherein the R of the uridine substrate is -NH₂, and the substrate base comprises one or more of 2-aminoadenine, 2-fluoroadenine, 2-chloroadenine, 2,6-dichloropurine, 2-amino-6-chloropurine, or 6-methoxyguanine.

4. The method of claim 1, wherein the pyrimidine nucleoside phosphorylase comprises an amino acid sequence as shown in SEQ ID NO: 1 and/or an amino acid sequence as shown in SEQ ID NO: 2; or
a protein having 70% or more identity to the amino acid sequence as shown in SEQ ID NO: 1 or SEQ ID NO:2 and having pyrimidine nucleoside phosphate activity;
wherein the purine nucleoside phosphorylase comprises an amino acid sequence as shown in SEQ ID NO: 3; or a protein having 70% or more identity to the amino acid sequence as shown in SEQ ID NO: 3 and having purine nucleoside phosphorylase activity.

5. The method of claim 2, wherein the R of the uridine substrate is -OH, and the pyrimidine nucleoside phosphorylase has an amino acid sequence as shown in SEQ ID NO: 2.

6. The method of claim 2, wherein the R of the uridine substrate is -H or -NH₂, and the pyrimidine nucleoside phosphorylase has the amino acid sequence as shown in SEQ ID NO: 1 and/or the amino acid sequence as shown in SEQ ID NO: 2.

7. The method of claim 2, wherein the R of the uridine substrate is -H, -NH₂ or -OH, and the purine nucleoside phosphorylase has the amino acid sequence as shown in SEQ ID NO: 3.

8. The method of claim 1, wherein the purine nucleoside comprises one or more of 2-fluoradenosine, 2-chloroadenosine, 2,6-dichloropurineside, 6-chloro-2-fluoradenosine, 6-chloroguanosine, 2-fluorodeoxyadenosine, 2-chlorodeoxyadenosine, 2,6-dichloropurine-2'-deoxyriboside, 6-chloro-2-fluoropurine-2'-deoxyriboside, 2-fluorodeoxyadenosine, 2-chlorodeoxyadenosine, 2,2'-diamino-2'-deoxyadenosine, 2-fluoro-2'-aminodeoxyadenosine, 2-chloro-2'-aminodeoxyadenosine, 2,6-dichloro-2'-aminodeoxyadenosine, 6-chloroguanine-2'-aminodeoxyadenosine, or 6-oxymethyl-2'-aminodeoxyguanosine.

9. The method of claim 1, wherein in the method, a concentration of the uridine substrate ranges from 1 to 5000 mM, and a concentration of the substrate base ranges from 1 mM to 5000 mM.

10. The method of claim 1, wherein a reaction time of the method ranges from 1h to 18 hrs.

11. The method of claim 1, wherein a reaction temperature of the method ranges from 40°C to 80 °C.
